(19) Europäisches Patentamt

European Patent Office

Office européen des brevets

(11) Publication number: **0 217 662 B1**

(12) **EUROPEAN PATENT SPECIFICATION**

(45) Date of publication of patent specification: **26.01.94** (51) Int. Cl.⁵: **A61L 2/12**

(21) Application number: **86307470.4**

(22) Date of filing: **30.09.86**

(54) **High temperature, short time heating method for sterilizing heat-sensitive biologicals fluids.**

(30) Priority: **30.09.85 US 782019**

(43) Date of publication of application:
**08.04.87 Bulletin 87/15**

(45) Publication of the grant of the patent:
**26.01.94 Bulletin 94/04**

(84) Designated Contracting States:
**CH DE FR GB IT LI SE**

(56) References cited:
EP-A- 0 015 055    EP-A- 0 094 611
DE-A- 1 814 084    DE-A- 3 233 282
FR-A- 2 542 009    US-A- 3 743 480

(73) Proprietor: **Charm, Stanley E.**
**21 Concolor Avenue**
**Newton Massachusetts 02158(US)**

(72) Inventor: **Charm, Stanley E.**
**21 Concolor Avenue**
**Newton Massachusetts 02158(US)**

(74) Representative: **Myerscough, Philip Boyd et al**
**J.A. Kemp & Co.**
**14 South Square, Gray's Inn**
**London WC1R 5LX (GB)**

# EP 0 217 662 B1

## Description

This invention relates to a method for heat pasteurizing or sterilizing heat-sensitive biological fluids.

It is often desirable, particularly in the food industry, to preserve heat-sensitive foods, such as milk or goods with delicate flavour components, by heating such heat-sensitive foods to high temperatures for very short periods of time, as in pasteurization and sterilization of food products. However, many such systems are available only for relatively large-scale food productions, and do not permit small-scale laboratory productions or experiments with valuable, low volume material, such as heat-sensitive biological fluids or suspension, used in the laboratory.

Further, it is often desirable to sterilize biological fluids or suspensions, such as plasma or protein containing fluids, to destroy selected pathogenic organisms, such as infectious agents like a virus or other agent composed substantially of protein and nucleic acids without destroying or substantially altering other microorganisms or precipitating or destroying other proteinaceous matter material. For example, it is desirable to destroy selectively virus and virus-type agents from blood plasma without clotting, clouding, aggregating, coagulating, precipitating or biologically altering the plasma in the process.

Methods for sterilizing biological materials by microwave energy are known (see for example DE-A-1814084, US-A-3743480 and EP-A-015055).

It is desirable to provide a continuous, fast, heat processing method for the high temperature, short time sterilization or pasteurization of heat-sensitive biological fluids and suspensions including body fluids, particularly for use with low volume biological fluids and for small-scale laboratory use.

The invention provides a method for the high temperature short time pasteurization or sterilization of a heat-sensitive biological fluid, said biological fluid or a mixture thereof with a dielectric enhancing additive having a dielectric constant of at least 100, which method comprises rapidly heating said fluid or mixture to a pasteurization or sterilization temperature by passing said fluid or mixture through tubing in a microwave chamber to expose it to microwave heating energy, holding the heated fluid or mixture at the pasteurization or sterilization temperature for not more than 1 second, cooling the heat pasteurized or sterilized fluid or mixture to a temperature of at most 40°C, and recovering a heat pasteurized or sterilized fluid or mixture, said heating being insufficient to alter or substantially destroy the biological activity of said heat sensitive biological fluid.

The method of the present invention permits the continuous rapid heating of biological fluids so as to effect sterilization or pasteurization without destroying or substantially altering biological activity, and is particularly useful for, but not limited to, small-scale laboratory production or experiments with valuable, low volume biological fluids or material and the selective destruction of infectious agents, like viruses and virus-type agents, from body fluids, such as blood plasma or serum.

In one embodiment, since the heating time in a microwave source depends on the dielectric constant of the biological fluid, a dielectric constant enhancing additive is typically employed and added to the heat-sensitive biological fluid to adjust the dielectric constant to allow for the short heat time period. The additive is of a type and is added in an amount sufficient to provide for enhanced dielectric constant of the fluid, so that the biological fluid may be rapidly heated by the microwave energy in the short time period. The dielectric constant additive should not affect the desired essential nature or quality of the biological fluid to which it is added, i.e. should be biologically inert. The additive may comprise a high dielectric salt or salt solution, and typically an inorganic metal salt, such as an alkali or alkaline earth salt, with sodium chloride, one preferred additive for blood plasma. Typically the additive is added to the biological fluids in an aqueous solution. Where the biological fluid already has a high dielectric constant, i.e. over 100, then depending on the heating time period desired, an additive need not be employed. A biological fluid containing the enhancing additive preferably has from 0.1 to 20 percent of a metal salt therein.

The biological fluids to which the dielectric constant enhancing additive is added is passed by through tubing, typically plastic or glass tubing, extending through the microwave oven, so that the fluid is rapidly heated to the selected sterilization or pasteurization temperature. The heated biological fluid with the additive is then cooled, and optionally all or substantially all of the dielectric additive is then removed and an aseptic biological fluid recovered.

The method may be used to sterilize a wide variety of microbiological fluids and suspensions, such as microbiological media, tissue culture media, suspensions that cannot be sterilized employing ultra-filtration, vaccines, mother's milk, etc. It may also be used to pasteurize or sterilize blood plasma (whole plasma or serum) and blood plasma products containing factors VIII and IX, and to destroy selectively agents like viruses, such as hepatitis or AIDS. The apparatus is designed to accommodate flow rates generally from about 3.0 liters per hour with a hold-up volume of about 0.4 liters or less.

2

The method employs a microwave oven and the necessary instrumentation to maintain sufficient back up pressure to permit a temperature in the oven, e.g. of 143°C, that is, selected sterilization or pasteurization temperature or such other predetermined temperature. Residence times at the sterilization temperature of 0.5 seconds or less at 143°C are typically sufficient to achieve sterility as defined by the 12 log cycle reductions of a heat resistant microorganism, such as Cl botulinum.

Since the heating-up time of the fluid in the microwave oven depends on the dielectric constant of the fluid being heated, the dielectric constant enhancing additive is added in various amounts as required, such as sodium chloride or other inert pharmaceutically inactive salt or salt solutions, more typically as a saline solution. While the amount of the dielectric constant enhancing additive may vary depending on the dielectric constant of the original biological fluid, generally from 0.1 to 10 percent or more by weight of the fluid of a salt may be added, more typically 0.5 to 5.0, and even more particularly 0.5 to 4.0 percent is often sufficient to enable rapid sterilization in a home or residential-type microwave oven. In a preferred embodiment blood plasma or serum contains up to 4 percent by weight of sodium chloride and is heated to a temperature sufficient to destroy viruses contained therein without destroying the clotting factors, in particular factors VIII and IX, of the plasma or serum, for example the blood plasma or serum is subjected to a temperature of 75°C or more for less than 0.5 seconds. Excessive quantities of the dielectric constant enhancing additive should be avoided, since optionally the additive should be removed from the sterilized biological fluid. The biological fluid with the additive may vary in dielectric constant depending on the desired temperature to be reached, but generally the biological fluid with the additive should have a dielectric constant of at least that of water, such as from 100 to 300, such as 100 to 200.

The size of the tubing in the microwave heater, usually in coiled or serpentine form, employed must hold up sufficient volume within the microwave chamber so that sufficient microwave energy will be absorbed to prevent burning out of the magnetron tubes in the microwave oven. A higher dielectric constant biological fluid will of course require a smaller hold-up volume than a lower dielectric constant material. Therefore, where adjustment of a dielectric constant cannot be entirely made employing a dielectric constant enhancing additive, varying tubing size for circulating the biological fluids through the microwave oven should be used to accommodate different ranges of dielectric constant fluids.

The biological fluid to which the dielectric constant enhancing additive has been added is generally circulated through tubing in the microwave heater by a pump, and a divert valve is employed between the microwave heater and the subsequent cooling mechanism, so that any biological fluid which does not reach the necessary selected sterilization temperature may be diverted. Generally such biological fluid is not recycled, but discarded, since high temperature heating of the biological fluid may alter the fluid. However, where such alteration does not affect the biological fluid, any biological fluid diverted by the divert valve and which is not adversely affected by reheating may be recycled as a biological fluid source for reheating in the microwave oven.

Also, a back pressure valve is typically employed after the cooler to prevent flashing of the fluid or vaporization of the heated biological fluid. Also, generally where the dielectric constant enhancing agent is added, it is desirable to remove such enhancing agent from the sterilized biological fluid under aseptic conditions, such as by the employment of ultrafiltration, dialysis or chromatography columns or other salt separation techniques. Thus, the dielectric constant enhancing agent, where applicable, should also be selected for easy or effective removal or separation from the sterilized biological fluid prior to recovery of the biological fluid in an aseptic receiver. Optionally, certain products, such as blood clotting factors, may be removed from blood plasma after the short time heating process and the removal of the dielectric enhancer, e.g. salt, may be unnecessary.

The heat processing apparatus used in the method of the invention comprises a high temperature, short time heat process apparatus for the heat sterilization of heat-sensitive biological fluids, which apparatus comprises a source of a heat-sensitive biological fluid to be sterilized, a source of a dielectric enhancing additive for addition in an amount to the source of the said heat-sensitive biological fluid to decrease the microwave heating time of the biological fluid, a microwave heating energy means to heat the fluid which contains the enhancing additive to a preselected sterilizing temperature in about one second or less, means to cool the heated sterilized biological fluid, and means to recover the heat sterilized biological fluid.

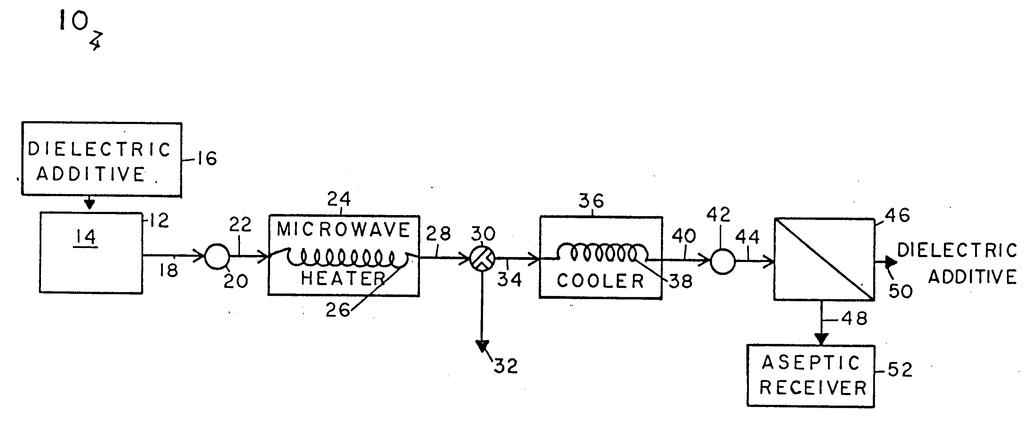

Embodiments of the present invention will now be described, by way of example, with reference to the accompanying drawing, the single figure of which is a schematic, illustrative drawing of a heat processing apparatus used in the method of the invention.

The drawing shows a heat processing system 10 comprising a container 12 containing a heat-sensitive biological fluid 14 and a source of a dielectric additive 16, such as a sodium chloride solution, which is added to the heat-sensitive biological fluid 14. The biological fluid 14 with the dielectric additive is then introduced through line 18 through pump 20 and line 22 into a microwave heater 24 wherein a defined

3

volume of the biological fluid is present in the coiled plastic tubing 26 and subject to microwave energy wherein the biological fluid now at a high dielectric constant is heated to 143°C for about one second or less, e.g. 0.1 to 0.5 seconds. The heat sterilized biological fluid is then withdrawn through line 28 through a divert three-way (off-on-divert) valve 30. If the biological fluid does not reach the heat sterilizing temperature (or in pasteurizing, the pasteurization temperature), the biological fluid is diverted through line 32 and discarded. The heat sterilized material is then introduced through line 34 into a coiled plastic tubing 38 in a cooler 36 where it is cooled to , for example, 40°C or below and then introduced through line 40 through a back pressure valve 42 which prevents the vapourization of the heated fluid. The heated fluid may then be introduced through line 44 into a separator 46, such as a dialysis unit or chromatography column, ultrafiltration or other separation means, and all or some of the added dielectric additive is then removed through line 50 and the sterilized biological fluid is removed through line 48 into an aseptic receiver 52 for laboratory, experimental or other use.

Example 1

Certain tests were conducted employing saline solutions of various weight percent salt with an initial temperature of about 27°C and a resulting cooled temperature of about 4°C employing the apparatus as described in the drawing with the test results as set forth in the accompanying table.

TABLE I

Saline Solution

| | Weight Percent Salt | Heating Time (seconds) to 143°C | Holding Time (seconds) at 143°C | Cooling Time (seconds) | Flow Rate L/hr |
|---|---|---|---|---|---|
| 1. | 0.5 | 16 | 0.5 | 2.5 | 3.6 |
| 2. | 0.9 | 6 | 0.5 | 2.5 | 3.3 |
| 3. | 1.5 | 5 | 0.5 | 2.5 | 3.3 |
| 4. | 4.0 | 3.5 | 0.15 | 2.5 | 3.3 |

As illustrated, the saline solutions of 0.9 and 1.5 percent in contrast to the lower dielectric constant saline solution of 0.5 percent provide for a very rapid increase in temperature in less than 6 seconds to the sterilization time and temperature of 143°C and for a holding time of one-half of a second, all with substantially the same flow rates. The use of a 4 percent saline solution provides for a more rapid temperature rise and short time, 0.15 seconds, at a sterilization temperature of 143-144°C.

## Example 2

A heat-sensitive biological fluid comprising blood plasma to which had been added 4 weight percent sodium chloride was processed in the apparatus of the drawing but without the removal of the salt, with the results shown in Table II.

TABLE II

(4 Percent Salt Blood Plasma Fluid)

|   | Final Temperature °C | Heating Time (seconds) | Holding Time (seconds) | Cooling Time (seconds) | Remarks |
|---|---|---|---|---|---|
| 1. | 68 | 1.5 | 0.05 | 0.9 | No clotting |
| 2. | 71 | 1.75 | 0.06 | 1.1 | No clotting |
| 3. | 76 | 1.9 | 0.07 | 1.2 | No clotting |
| 4. | 81 | 2.1 | 0.07 | 1.3 | Clotting |

Flow Rate: 3.3 L/hr.

Microwave oven power: 700 watts.

Hold up volume in microwave: 40 ml.

6

In Example 2 there was no change in albumin, globins, or factors VIII and IX in comparison to an unprocessed control sample. Example 2 demonstrates that heat-sensitive blood plasma containing pathogenic viruses, such as hepatitis B or AIDS, may be pasteurized with the destruction of the viruses by the rapid high temperature microwave heating method. As illustrated, the pasteurization holding time with the addition of the dielectric additive is very short, 0.05 to .07 seconds, to provide heating without affecting clotting factors unless the heating time is more than 1.9 seconds with a holding time of 0.07 seconds.

Example 3

Blood plasma has been processed in the prior art at temperatures of 59°C for a time period of 12 hours in an attempt to destroy viruses and yet to preserve the blood clotting factors of the blood plasma, e.g. factors VIII and IX; however, the hepatitis B virus and other agents can survive this process. Further, the process is time consuming. It has been found possible to achieve high temperatures, e.g. 75°C or more, for short time periods, e.g. for 0.5 seconds or less, such as 0.05 seconds, employing microwave energy and still preserve the blood clotting factors in the blood plasma while destroying by the short heating time infectious agents, such as viruses. By achieving temperatures and times in the range of 75°C for 0.05 seconds, it is possible to preserve factors VIII and IX in blood plasma with 4 percent salt in the plasma and to destroy viruses in the plasma.

In determining the amount of dielectric additive necessary to be added, a measure of the dielectric constant may be obtained by passing the liquid at a rate of 8.35 liters/hour through 8.5 m (28 feet) of 0.16 cm internal diameter tubing spaced throughout the volume of the microwave heater and allowing the system to come to a steady state. The liquid residence time in the microwave is 7.04 seconds.

TABLE III

| | Material | Initial Temperature (C) | Final Temperature (C) | Difference (C) | Percent Change From Water |
|---|---|---|---|---|---|
| 1. | Water | 25.6 | 62.2 | 36.6 | --- |
| 2. | 0.5% Salt | 25.0 | 72.8 | 47.8 | +30.6 |
| 3. | 1.0% Salt | 22.8 | 78.3 | 55.5 | +51.6 |
| 4. | 2% Salt | 23.9 | 85.0 | 61.1 | +66.9 |
| 5. | 4% Salt | 25.0 | 91.7 | 66.7 | +82.2 |
| 6. | 10% Salt | 28.3 | 98.3 | 70.0 | +91.3 |
| 7. | 20% Salt | 22.2 | 95 | 72.7 | +98.6 |

As illustrated by the test data in Table III, the increase in temperature rise is associated with an increase in dielectric constant (water having a dielectric constant of about 69, and 4 percent salt solutions about 126).

**Claims**

1. A method for the high temperature short time pasteurization or sterilization of a heat-sensitive biological fluid, said biological fluid or a mixture thereof with a dielectric enhancing additive having a dielectric constant of at least 100, which method comprises rapidly heating said fluid or mixture to a pasteurization or sterilization temperature by passing said fluid or mixture through tubing in a microwave chamber to expose it to microwave heating energy, holding the heated fluid or mixture at the pasteurization or sterilization temperature for not more than 1 second, cooling the heat pasteurized or sterilized fluid or mixture to a temperature of at most 40° C, and recovering a heat pasteurized or sterilized fluid or mixture, said heating being insufficient to alter or substantially destroy the biological activity of said heat-sensitive biological fluid.

2. A method according to claim 1, which includes separating all or substantially all of the dielectric enhancing additive from the heat pasteurized or sterilized biological fluid.

3. A method according to claim 1 or 2, wherein the biological fluid containing the enhancing additive has from 0.1 to 20 percent of a metal salt therein.

4. A method according to claim 1, 2 or 3, wherein the biological fluid comprises blood plasma or serum.

5. A method according to claim 4, wherein the blood plasma or serum contains up to 4 percent by weight of sodium chloride and is heated to a temperature sufficient to destroy viruses contained therein without destroying the clotting factors of the plasma or serum.

6. A method according to claim 5, wherein the blood plasma or serum contains blood clotting factors VIII and IX.

7. A method according to claim 6, wherein the blood plasma or serum subjected to a temperature of 75° C or more for less than 0.5 seconds.

**Patentansprüche**

1. Ein Verfahren für die Hochtemperatur-Kurzzeitpasteurisierung oder -sterilisierung eines wärmeempfindlichen biologischen Fluids, wobei das biologische Fluid oder eine Mischung hiervon einen dielektrisch verstärkenden Zusatzstoff mit einer Dielektrizitätskonstante von wenigstens 100 besitzt, wobei das Verfahren die schnelle Erhitzung des Fluids oder des Gemischs auf eine Pasteurisierungs- oder Sterilisierungstemperatur, indem das Fluid oder das Gemisch durch ein Rohrleitungssystem in eine Mikrowellenkammer geschickt wird, um es mit Mikrowellen-Heizenergie zu beaufschlagen, das Halten des erhitzten Fluids oder des erhitzten Gemischs auf der Pasteurisierungs- oder Sterilisierungstemperatur für nicht mehr als 1 Sekunde, das Abkühlen des durch Hitze pasteurisierten oder sterilisierten Fluids oder Gemischs auf eine Temperatur von höchstens 40°C sowie die Wiederherstellung eines durch Hitze pasteurisierten oder sterilisierten Fluids oder Gemischs enthält, wobei die Erhitzung nicht ausreicht, um die biologische Aktivität des wärmeempfindlichen biologischen Fluids zu verändern oder wesentlich zu zerstören.

2. Ein Verfahren gemäß Anspruch 1, das die Abtrennung des gesamten oder im wesentlichen des gesamten dielektrisch verstärkenden Zusatzstoffs von dem durch Hitze pasteurisierten oder sterilisierten biologischen Fluid enthält.

3. Ein Verfahren gemäß Anspruch 1 oder 2, bei dem das biologische Fluid, das den verstärkenden Zusatzstoff enthält, 0,1 bis 20 Prozent eines metallischen Salzes enthält.

4. Ein Verfahren gemäß Anspruch 1, 2 oder 3, bei dem das biologische Fluid Blutplasma oder -serum enthält.

5. Ein Verfahren gemäß Anspruch 4, bei dem das Blutplasma oder -serum bis zu 4 Gewichtsprozent Natriumchlorid enthält und auf eine Temperatur erhitzt wird, die ausreicht, um darin enthaltene Viren zu zerstören, ohne die Gerinnungsfaktoren des Plasmas oder des Serums zu zerstören.

**EP 0 217 662 B1**

**6.** Ein Verfahren gemäß Anspruch 5, bei dem das Blutplasma oder -serum Blutgerinnungsfaktoren VIII und IX enthält.

**7.** Ein Verfahren gemäß Anspruch 6, bei dem das Blutplasma oder -serum für weniger als 0,5 Sekunden einer Temperatur von 75°C oder mehr unterworfen wird.

**Revendications**

**1.** Procédé de pasteurisation ou de stérilisation, pendant un temps bref, à haute température, d'un fluide biologique sensible à la chaleur, ledit fluide biologique ou son mélange avec un additif augmentant la constante diélectrique présentant une constante diélectrique d'au moins 100, procédé qui comprend le chauffage rapide dudit fluide ou mélange à une température de pasteurisation ou de stérilisation en faisant passer ledit fluide ou mélange à travers un tube dans une chambre à micro-ondes pour l'exposer à l'énergie chauffante de micro-ondes, le maintien du fluide ou du mélange chauffé à la température de pasteurisation ou de stérilisation pendant un temps ne dépassant pas 1 seconde, le refroidissement du fluide ou du mélange pasteurisé ou stérilisé par la chaleur jusqu'à une température au plus égale à 40°C, et la récupération d'un fluide ou d'un mélange pasteurisé ou stérilisé par la chaleur, ledit chauffage étant insuffisant pour modifier ou pratiquement détruire l'activité biologique dudit fluide biologique sensible à la chaleur.

**2.** Procédé selon la revendication 1, qui comprend la séparation de tout ou de pratiquement tout l'additif augmentant la constante diélectrique du fluide biologique pasteurisé ou stérilisé par la chaleur.

**3.** Procédé selon la revendication 1 ou 2, dans lequel le fluide biologique contenant l'additif augmentant la constante diélectrique contient de 0,1 à 20 pourcent d'un sel métallique.

**4.** Procédé selon la revendication 1, 2 ou 3, dans lequel le fluide biologique comprend du sérum ou plasma sanguin.

**5.** Procédé selon la revendication 4, dans lequel le sérum ou plasma sanguin contient jusqu'à 4 pourcent en poids de chlorure de sodium et dans lequel on le chauffe à une température suffisante pour détruire les virus qu'il contient sans détruire les facteurs coagulants du sérum ou plasma.

**6.** Procédé selon la revendication 5, dans lequel le sérum ou plasma sanguin contient des facteurs coagulants le sang VIII et IX.

**7.** Procédé selon la revendication 6, dans lequel on soumet le sérum ou plasma sanguin à une température de 75°C ou plus pendant moins de 0,5 seconde.

10

EP 0 217 662 B1

$10$

| DIELECTRIC ADDITIVE |—16

14 —12

18 → 20 → 22

MICROWAVE HEATER (24, 26) —28

30 —34 → 32

COOLER (36, 38) —40

42 —44

46 → DIELECTRIC ADDITIVE —50

—48

| ASEPTIC RECEIVER |—52